Europäisches Patentamt

European Patent Office

Office européen des brevets

(19)

(11) Numéro de publication : **0 477 076 A1**

# (12) DEMANDE DE BREVET EUROPEEN

(21) Numéro de dépôt : **91402448.4**

(51) Int. Cl.⁵ : **A61F 2/36**

(22) Date de dépôt : **13.09.91**

(30) Priorité : **19.09.90 FR 9011532**

(43) Date de publication de la demande :
**25.03.92 Bulletin 92/13**

(84) Etats contractants désignés :
**AT BE CH DE DK ES FR GB GR IT LI LU NL SE**

(71) Demandeur : **LA CELLULOSE DU PIN**
**353, bd du Président Wilson**
**F-33200 Bordeaux (FR)**

(72) Inventeur : **Pommier, Jean-Claude**
**35 Allée de Gascogne**
**F-33170 Gradignan (FR)**
Inventeur : **Poustis, Joel**
**3 Allée de Saintonge**
**Cap de Bos, F-33600 Pessac (FR)**
Inventeur : **Bacquey, Charles**
**33 Rue du Médoc le Haillan**
**F-33160 St Medard en Jalles (FR)**
Inventeur : **Chauveaux, Dominique**
**13 Rue Marceaux**
**F-3300 Bordeaux (FR)**

(74) Mandataire : **Muller, René et al**
**SAINT-GOBAIN RECHERCHE 39, quai Lucien**
**Lefranc-BP 135**
**F-93303 Aubervilliers Cédex (FR)**

(54) **Système de montage pour des prothèses articulaires.**

(57) L'invention concerne un système de montage pour une prothèse articulaire ou pièce orthopédique 1 dont une partie 4 est destinée à être scellée dans le canal médullaire d'un os prothésé 11 à l'aide d'une gaine d'ajustage 10, ce système comprenant une prothèse munie de moyens 7, 8 permettant de libérer un espace entre les surfaces en contact de la gaine d'ajustage 10 et de la partie scellée 4 de la prothèse.
Le système de montage selon l'invention utilise le concept dit à scellement réversible mettant en jeu un matériau hydrophyle bio-compatible doué d'un potentiel d'expansion volumique.

EP 0 477 076 A1

Fig. 2

La présente invention concerne un système de montage pour des prothèses articulaires et en particulier un système de montage pour des prothèses ou pièces orthopédiques utilisant un concept à scellement réversible.

La publication de brevet européen EP 0 256 906 décrit un nouveau concept de scellement d'une prothèse articulaire. Ce scellement dit réversible utilise un matériau bio-compatible présentant un potentiel d'expansion volumique susceptible de créer des efforts normaux aux surfaces en regard et qui s'opposent aux mouvements relatifs de la pièce prothétique et de l'os prothésé. Ainsi, dans l'application au scellement d'une prothèse articulaire de hanche, le matériau bio-compatible en forme d'une gaine d'ajustage pour la queue de prothèse, grâce à son potentiel de reprise en eau entrainant une expansion volumique, fournit un scellement résistant de la queue de prothèse dans la cavité fémorale.

Pour retirer la prothèse, en cas de rupture de celle-ci ou pour d'autres raisons, il suffit de fournir par exemple un supplément d'eau artificiellement à la gaine tout en libérant un espace d'expansion volumique pour cette gaine. La reprise en eau supplémentaire entraine une perte des propriétés de résistance mécanique de la gaine, ce qui facilite le retrait de la prothèse.

Un des problèmes posés par ce type de scellement est la conception d'une structure de prothèse autorisant une modification du volume de la gaine suffisante pour le désancrage de la queue de prothèse au moment désiré.

L'invention fournit une solution à ce problème. Elle propose un système de montage pour prothèse articulaire selon le concept à scellement réversible, cette prothèse pouvant être facilement descellée et retirée en cas de besoin. Le système de montage selon l'invention comprend une prothèse munie de moyens permettant de libérer un espace entre les surfaces en contact de la gaine d'ajustage et de la queue ou partie scellée de la prothèse et une gaine d'ajustage en un matériau biocompatible à base de cellulose purifiée, doué d'un potentiel d'expansion volumique ou de retrait volumique selon qu'on ajoute ou retire de l'eau ou un liquide physiologique audit matériau.

Sous un des aspects de l'invention, les moyens dont est munie la prothèse sont tout ou partie de moyens permettant de chauffer la gaine d'ajustage de façon à contracter le matériau et par là de libérer un espace permettant le retrait de la queue de prothèse.

Les moyens permettant de chauffer la gaine d'ajustage sont par exemple le corps même de la queue de prothèse qui à cette fin est réalisé en un matériau pouvant être chauffé par micro-ondes par exemple. Ce peut être un ou plusieurs fils de résistance électrique incorporés à la queue de la prothèse et pouvant être connectés à une source de tension adéquate.

Dans une autre variante, les moyens de chauffage de la queue de prothèse peuvent comprendre une couche de résistance électrique recouvrant au moins partiellement cette queue de prothèse.

Sous un autre aspect de l'invention, la prothèse comprend des moyens permettant de réduire le volume de sa partie scellée de façon à libérer un espace périphérique pour permettre une expansion du matériau de scellement bio-compatible formant la gaine d'ajustage, cette expansion étant accompagnée d'une perte de propriétés mécaniques, ce qui finalement permet l'extraction d'une part de la queue de prothèse et d'autre part de la gaine d'ajustage.

Sous une forme de réalisation de la prothèse, celle-ci comporte une queue comprenant une tige centrale montée notamment par vissage dans un alésage déformable tel une cheville métallique.

Pour la mise en place de la prothèse, la tige est au préalable vissée dans la cheville et la queue occupe alors un volume maximum. Lorsqu'on désire, le cas échéant, retirer la prothèse, il suffit alors de dévisser la tige centrale, ce qui ramène la cheville ou queue à un volume plus réduit. La réduction de volume de la queue de prothèse libère ainsi un espace autorisant une expansion de la gaine d'ajustage accompagnée si nécessaire d'une perte de propriétés mécaniques facilitant l'extraction de la prothèse et de la gaine.

Sous une autre forme de réalisation de la prothèse, celle-ci présente une queue dont des éléments ou parties périphériques sont constituées d'éléments extractibles et pouvant être retirés séparément d'une partie fixe. Ainsi, par exemple, la queue de prothèse peut présenter des cannelures ou rainures périphériques, occupées par des tiges ou des vis pouvant être extraites facilement le cas échéant pour libérer ainsi des espaces périphériques. Ces espaces périphériques libérés permettent alors une expansion de la gaine facilitant comme précédemment l'extraction de la queue de prothèse et de la gaine.

Comme décrit précédemment, la queue de prothèse peut être formée de plusieurs parties facilitant son montage et son retrait ou désancrage.

La gaine assurant le scellement réversible présente la forme et les dimensions adaptées d'une part à la queue de prothèse et d'autre part aux caractéristiques de l'os prothésé. Elle doit permettre un assemblage aisé. Elle peut être obtenue par usinage à la forme et aux dimensions requises.

Avantageusement, la gaine assurant le scellement réversible de la queue de prothèse peut aussi être réalisée en plusieurs parties, par exemple deux ou trois coquilles.

Le matériau formant la gaine est un matériau à base de cellulose purifiée, matériau duquel on a retiré par séchage la totalité ou la majeure partie de l'eau, et qui présente un potentiel de reprise d'eau modulable jusqu'à une teneur en eau de 60 % environ. Ce matériau et un procédé de fabrication est décrit dans la publication de brevet EP 0 256 906 dont la description est incorporée ici par cette référence.

D'autres caractéristiques et avantages de l'invention apparaitront dans la description suivante d'exemples de réalisation selon l'invention.

La figure 1 représente une queue de prothèse de hanche qui, selon l'invention est munie de moyens permettant de réduire son volume afin de libérer un espace périphérique.

La figure 2 représente le système constitué par la queue de prothèse et sa gaine d'ajustage pour son scellement, le tout monté dans l'os prothésé.

La figure 3 représente le système de la figure 2, dans la position d'extraction, la queue de prothèse présentant un volume réduit facilitant son extraction de la cavité osseuse.

La figure 4 représente une variante de réalisation d'une queue de prothèse de hanche.

La figure 5 est une vue en section en perspective de la queue de prothèse selon la section A-A.

La figure 6 est une vue en section de la queue de prothèse munie de sa gaine d'ajustage.

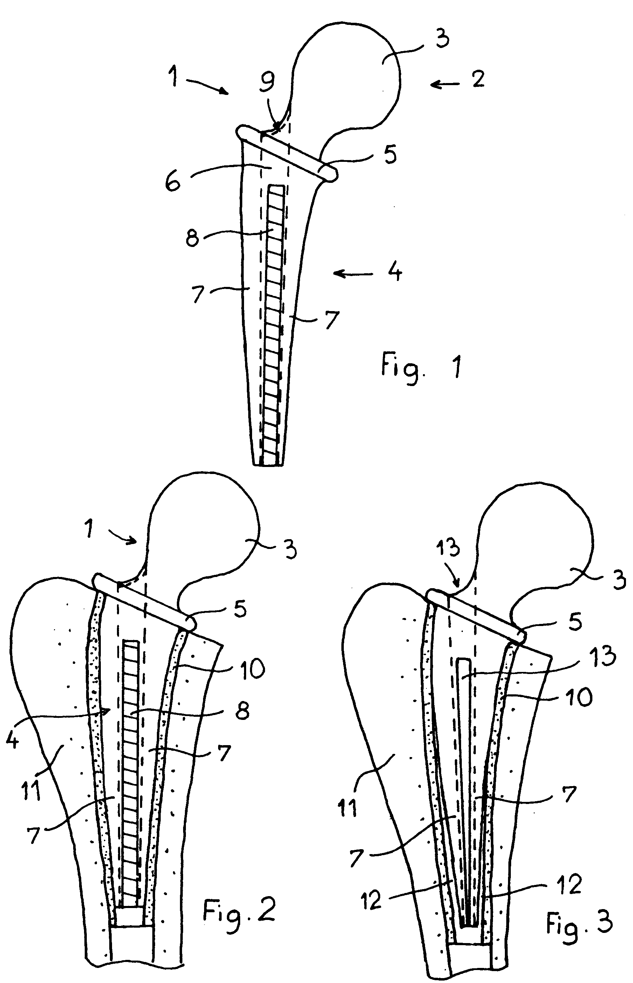

La queue de prothèse 1 de hanche représentée schématiquement sur la figure 1, comprend plusieurs parties. La partie supérieure 2 présente une extrémité 3 de forme arrondie nécessaire à l'articulation et aux mouvements en rotation dans la partie réceptrice (non représentée) solidaire de la hanche. La partie inférieure 4 est destinée à être scellée dans le canal médullaire de l'os. Cette partie inférieure 4 est séparée de la partie supérieure 2 par une collerette 5 destinée à empêcher l'expansion à ce niveau de la gaine d'ajustage décrite ci-après, et elle est formée d'une partie annulaire 6 à partir de laquelle partent plusieurs brins 7, par exemple 4 brins s'étendant sur essentiellement toute la longueur de cette partie inférieure et qui sont maintenus séparés par une tige centrale filetée 8. L'action de la tige centrale 8 sur la partie inférieure est celle d'une vis dans une cheville : par vissage, elle écarte les brins 7. Lorsqu'elle est entièrement vissée, la partie inférieure occupe un volume maximum.

Toutes les parties de la queue de prothèse peuvent être en acier inoxydable ou en un autre métal ou matériau adéquat, étant entendu que la nature du matériau et la structure des brins doivent procurer à la queue de prothèse une élasticité suffisante pour l'écartement des brins lors du vissage de la tige centrale, et leur rapprochement lors du dévissage et du retrait de cette tige. Pour visser ou dévisser la tige centrale, une encoche 9 peut être prévue dans le haut de la tige.

Sur la figure 2, la queue de prothèse 1 est représentée munie de sa gaine d'ajustage 10 assurant le scellement dans la cavité fémorale de l'os 11. Le scellement de la queue de prothèse est assuré par une expansion volumique de la gaine, due à une reprise en eau du matériau qui au préalable avait été séché.

La gaine est en un matériau à base de cellulose purifiée et elle est fabriquée selon la méthode décrite dans la publication de brevet européen EP-A-0 256 906 déjà cité.

Elle peut être formée d'une seule pièce obtenue par usinage ou moulage. Elle peut aussi être formée par un assemblage de plusieurs coquilles généralement deux ou trois.

La gaine est confectionnée à partir d'un matériau obtenu par la mise en oeuvre du procédé décrit dans la publication du brevet européen EP-A-0 256 906 cité ci-dessus, et qui comprend les opérations suivantes :

- on prépare de l'alcali-cellulose par trempage de la pâte de cellulose notamment de la pâte obtenue par le procédé bisulfite acide, dans une solution de soude suivi d'un essorage et d'un pressage pour atteindre un poids en cellulose sèche compris entre 30 % et 40 % environ,

- on réalise ensuite la sulfuration de l'alcali-cellulose par du sulfure de carbone, de préférence en excès par rapport aux quantités stoéchiométriques, pour obtenir du xanthate de sodium,

- on dissous le xanthate de sodium dans une solution de soude diluée pour obtenir de la viscose, c'est-à-dire une solution contenant de la cellulose et de la soude à des teneurs bien déterminées, de préférence de 7 à 12 % en poids de cellulose et de 4 à 8 % en poids de soude,

- on soumet la solution obtenue à un traitement thermique afin de provoquer la coagulation de la viscose et à un traitement d'élimination du soufre, ces deux traitements s'effectuant par phases de façon alternée, pour obtenir un matériau amorphe et présentant un caractère caoutchouteux,

- et on sèche le matériau obtenu.

L'élimination du soufre s'effectue par lavage avec de l'eau et un produit réactif avec le soufre, notamment le sulfite de sodium, le carbonate de sodium.

Le matériau finalement obtenu à la consistance du bois et il présente une teneur en soufre inférieure à 0,1 % en poids.

La forme et les dimensions de la gaine sont adaptées aux caractéristiques de la queue de prothèse et à celles de l'os prothésé.

Le scellement réalisé après une reprise en eau contrôlée s'oppose à tous les mouvements relatifs de la prothèse et de la pièce osseuse réceptrice.

Lorsqu'il est nécessaire pour une raison ou une autre de retirer la prothèse, on dévisse et on retire la tige filetée 8, ce qui provoque le rapprochement des brins 7 comme représenté sur la figure 3 et la libération d'un espace périphérique 12 entre la queue de prothèse et la gaine. Par injection d'eau ou de liquide physiologique par le trou 13 laissé par le retrait de la tige filetée, on apporte au matériau de la gaine un supplément d'eau qui permet une expansion de ce matériau, liée à une perte de ses caractéristiques mécaniques facilitant le retrait de la queue de prothèse et de la gaine de scellement.

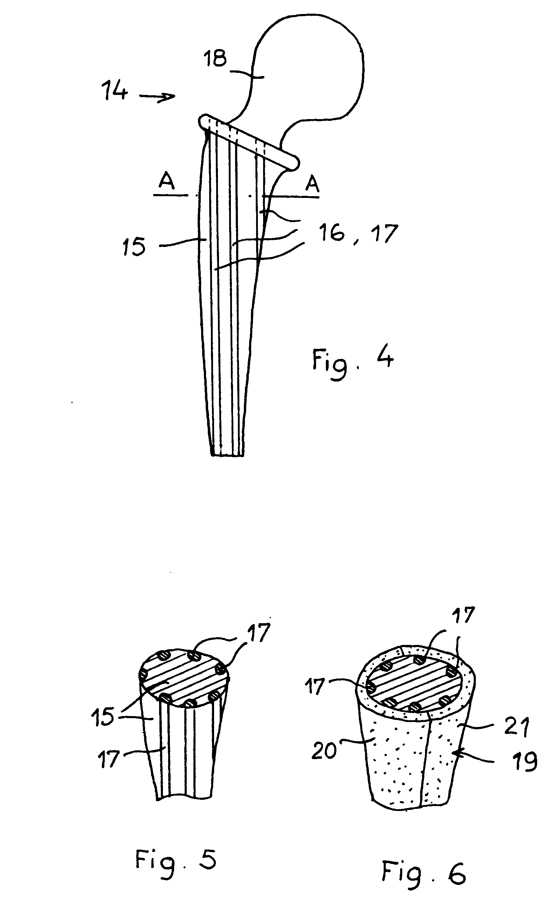

Sur les figures 4 et 5, on a représenté une variante de réalisation d'une queue 14 de prothèse de hanche. Dans cette variante, la partie inférieure 15 de la queue 14 présente des cannelures, ou gorges 16, ou rainures périphériques comblées par des tiges filetés ou non 17 pouvant être extraites le cas échéant par dévissage ou coulissement vers l'extrémité supérieure 18 de la queue.

Cette prothèse comme dans le cas de la variante précédente est scellée dans la partie supérieure du canal médullaire du fémur par l'utilisation d'une gaine en matériau bio-compatible doué d'un potentiel d'expansion par absorption d'eau ou de liquide physiologique. La figure 5 est une section en perspective de la queue selon la ligne de découpe A-A.

La figure 6 est une section de la queue munie de sa gaine d'ajustage 19 formée ici de deux coquilles 20, 21.

Selon la forme en section des tiges, la queue de prothèse peut présenter une ligne périphérique canelée ou plus régulière.

Pour desceller la queue de prothèse, il suffit de retirer par coulissement les tiges 17 de leur compartiment en forme de gorges 16.

Les espaces périphériques formés par les gorges vides dues au retrait des tiges 17 vont autoriser une expansion volumique de matériaux constituant la gaine lors d'un apport d'eau ou de liquide physiologique, cet apport pouvant se faire par le haut des gorges 16.

Une variante de la queue de prothèse de hanche du système selon l'invention utilisant cette fois des moyens permettant de chauffer la gaine d'ajustage de façon à contracter le matériau constituant cette gaine peut présenter une structure semblable à celle décrite en relation avec les figures 4, 5 et 6. Dans ce cas les tiges 17 sont des fils de résistances électriques. Ces fils restent bien entendu en place lors de l'opération de descellement de la queue de prothèse. Pour opérer ce descellement, on connecte les fils de résistance à une source de tension électrique adéquate. Par chauffage de ces fils, le matériau constituant la gaine d'ajustage se contracte et libère ainsi la queue de prothèse.

**Revendications**

1. Ensemble de montage comprenant une prothèse ou pièce orthopédique (1, 14) dont une partie (4, 15) est destinée à être scellée dans le canal médullaire d'un os prothésé (11) et une gaine d'ajustage (10, 19) pour la partie destinée a être scellée, caractérisé en ce que la prothèse comprend des moyens (7, 8, 16, 17) permettant de libérer un espace entre les surfaces en contact de la gaine d'ajustage et de la partie scellée de la prothèse, la gaine d'ajustage (10, 19) étant formée d'une ou plusieurs parties en un matériau bio-compatible doué d'un potentiel d'expansion volumique ou de retrait volumique, selon qu'on ajoute ou retire de l'eau ou un liquide physiologique.

2. Ensemble selon la revendication 1, caractérisé en ce que les moyens (7, 8, 16, 17) sont tout ou partie de moyens permettant de chauffer la gaine d'ajustage.

3. Ensemble selon la revendication 2, caractérisé en ce que les moyens permettant de chauffer sont au moins un fil ou une couche de résistance électrique.

4. Ensemble selon la revendication 1, caractérisé en ce que les moyens permettant de libérer un espace sont des moyens permettant de réduire le volume de la partie scellée.

5. Ensemble selon la revendication 4, caractérisé en ce que la prothèse comporte une queue (4) comprenant une tige centrale (8) montée dans un alésage déformable (6, 7).

6. Ensemble selon la revendication 5, caractérisé en ce que la tige centrale est une vis (8) écartant en position vissée, ou rapprochant en position dévissée des éléments (7) de l'alésage.

7. Ensemble selon la revendication 4, caractérisé en ce que la partie (15) destinée à être scellée présente des éléments périphériques extractibles (17).

8. Ensemble selon la revendication 7, caractérisé en ce que la partie (15) destinée à être scellée présente des gorges (16) occupées par des tiges ou des vis (17).

Fig. 1

Fig. 2

Fig. 3

Fig. 4

Fig. 5

Fig. 6

**Office européen des brevets**

# RAPPORT DE RECHERCHE EUROPEENNE

Numero de la demande

EP 91 40 2448

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | US-A-4 888 022 (HUEBSCH) | 1 | A61F2/36 |
| Y | * abrégé; figures 1,2 * | 2-6 | |
| | * colonne 5, ligne 20 - colonne 6, ligne 28 * | | |
| | --- | | |
| X | FR-A-2 629 337 (BIGAN ET AL.) | 1 | |
| | * abrégé; figures * | | |
| | --- | | |
| Y | FR-A-2 344 274 (KRAUS) | 2,3 | |
| | * revendication 1; figures 1-4 * | | |
| | --- | | |
| Y | US-A-4 011 602 (RYBICKI ET AL.) | 4-6 | |
| | * abrégé; figures 2,3 * | | |
| | --- | | |
| A | DE-A-3 613 657 (LAABS) | 7,8 | |
| | * figure 3 * | | |
| | ----- | | |

**DOMAINES TECHNIQUES RECHERCHES (Int. Cl.5 )**

A61F

Le présent rapport a été établi pour toutes les revendications

| Lieu de la recherche | Date d'achèvement de la recherche | Examinateur |
|---|---|---|
| LA HAYE | 16 DECEMBRE 1991 | SANCHEZ Y SANCHEZ J. |

EPO FORM 1503 03.82 (P0402)